(19) Europäisches Patentamt
European Patent Office
Office européen des brevets

(11) EP 1 240 899 A2

(12) # EUROPEAN PATENT APPLICATION

(43) Date of publication:
**18.09.2002 Bulletin 2002/38**

(51) Int Cl.[7]: **A61K 31/7056**, A61K 38/21, A61P 31/14

(21) Application number: **02009781.2**

(22) Date of filing: **21.01.1997**

(84) Designated Contracting States:
**AT BE CH DE DK ES FI FR GB GR IE IT LI LU MC NL PT SE**

(30) Priority: **23.01.1996 US 590449**
**14.01.1997 US 36094**

(62) Document number(s) of the earlier application(s) in accordance with Art. 76 EPC:
**97904765.1 / 0 879 056**

(71) Applicant: **ICN Pharmaceuticals, Inc.**
**Costa Mesa, CA 92626 (US)**

(72) Inventors:
- **Tam, Robert**
  **Costa Mesa, CA 92626 (US)**
- **Ramasamy, Kandsamy**
  **Laguna Hills, CA 92653 (US)**
- **Averett, Devron**
  **Irvine, CA 92612 (US)**

(74) Representative: **Viering, Jentschura & Partner**
**Postfach 22 14 43**
**80504 München (DE)**

Remarks:
This application was filed on 30 - 04 - 2002 as a divisional application to the application mentioned under INID code 62.

(54) **Modulation of Th1/Th2 Cytokine expression by Ribavirin and Ribavirin analogs in actived T-Lymphocytes for treating HCV infections**

(57) Ribavirin® is administered to a patient in a dosage range which is effective to modulate lymphokine expression in activated T cells. In particular, Ribavirin® is used to suppress Th2-mediated T cell responses and promote Th1-mediated T cell response. In other apsects of the invention, one or more Ribavirin® analogs are administered to a patient in a dosage range which is effective to modulate lymphokine expression in activated T cells. The Ribavirin® analog(s) may be used to suppress or enhance Th1 or Th2-mediated T cell responses.

EP 1 240 899 A2

## Description

FIELD OF THE INVENTION

The field of the invention is immunology.

BACKGROUND OF THE INVENTION

The lymphokines arc a group of polypeptides belonging to the family of cytokines, i.e. hormone-like molecules that can affect various cell functions and enable communication between different cells. Recent developments have helped to clarify the role of lymphokines in be immune response. Lymphokine production by helper $CD4^+$ (and also in $CD8^+$) T cells frequently fall into one of two phenotypes, Th1 and Th2, in both murine and human systems (Romagnani, 1991, *Immunol Today* **12:** 256-257, Mosmann, 1989, *Annu Rev Immunol*, **7**: 145-173). Th1 cells produce interleukin 2 (IL-2), tumor necrosis factor (TNF$\alpha$) and interferon gamma (IFN$\gamma$) and they are responsible primarily for cell-mediated immunity such as delayed type hypersensitivity. Th2 cells produce interleukins, IL-4, IL-5, IL-6, IL-9, IL-10 and IL-13 and are primarily involved in providing optimal help for humoral immune responses such as IgE and IgG4 antibody isotype switching (Mosmann, 1989, *Annu Rev Immunol*, **7**: 145-173).

Strongly polarized Th1 and Th2 responses not only play different roles in protection, they can promote different immunopathological reactions. Th1-type responses are involved organ specific autoimmunity such as experimental autoimmune uveoretinitis (Dubey et al, 1991, *Eur Cytokine Network* **2** : 147-152), experimental autoimmune encephalitis (EAE) (Beraud et al, 1991, *Cell Immunol* **133** : 379-389) and insulin dependent diabetes mellitus (Hahn et al, 1987, *Eur J Immunol* **18** : 2037-2042), in contact dermatitis (Kapsenberg et al, *Immunol Today* **12** : 392-395), and in some chronic inflammatory disorders. In contrast Th2-type responses are responsible for triggering allergic atopic disorders (against common environmental allergens) such as allergic asthma (Walker et al, 1992, *Am Rev Resp Dis* **148** : 109-115) and atopic dermatitis (van der Heijden et al, 1991, *J Invest Derm* **97** : 389-394), are thought to exacerbate infection with tissue-dwelling protozoa such as helminths (Finkelman et al, 1991, *Immunoparasitol Today* **12** : A62-66) and Leishmania major (Caceres-Dittmar et al, 1993, *Clin Exp Immunol* **91**: 500-505), are preferentially induced in certain primary immunodeficiencies such as hyper-IgE syndrome (Del Prete et al, 1989, *J Clin Invest* **84**: 1830-1835) and OmennÕs syndrome (Schandene et al, 1993, *Eur J Immunol* **23**: 56-60), and are associated with reduced hyper-IgE syndrome (Del Prete et al, 1989, *J Clin Invest* **84**: 1830-1835) and OmennÕs syndrome (Schandene et al, 1993, *Eur J Immunol* **23**: 56-60), and are associated with reduced ability to suppress HIV replication (Barker et al, 1995, *Proc Soc Nat Acad Sci USA* **92**: 11135-11139).

Thus, it is clear that modulation of the lymphokine profiles of the aforementioned disease states would be of therapeutic benefit. Promoting a Th1 response would most likely lead to the reversal of a Th2 phenotype and vice versa. Monoclonal antibodies (mAb) to lymphokines, lymphokines themselves and other agents such as thiol antioxidants (Jeannin et al, 1995, *J Exp Med* **182**: 1785 - 1792) have been shown to reverse the pathogenesis of certain diseases by inhibiting the disease-promoting cytokine pattern, either Th1 or Th2. For example, intracellular protozoan infections are limited by IFN$\gamma$ but exacerbated by IL-4, while nematode infections are controlled by IL-4 and exacerbated by IFN$\gamma$ (Heinzel et al, 1989, *J Exp Med* **162**: 59-72, Else et al, 1994, *J Exp Med* **179**: 347-351). Insulin-dependent diabetes mellitus in NOD mice and EAE in mice and rats can be ameliorated by treatment with IL-4 or anti- IFN$\gamma$ mAb before development of the disease (Rapoport et al, *1993, J Exp Med* **178**: 87-99, Racke et al, 1994, *J Exp Med* **180**: 1961-1966, Campbell et al, *1991*, *J Clin Invest* **87**: 739-742). In addition, autoimmune graft versus host disease (GVHD) that is characterized by a systemic lupus erythrematosus-like syndrome is associated with Th2 lymphokine production and is inhibited by anti-IL-4 antibody (Umland et al, 1992, *Clin Immunol Immunopathol* **63:** 66-73). On the other hand, Th1 cytokines are produced in acute GVHD, in which donor $CD8^+$ T cells develop into CTL and destroy the host immune system. Treatment with anti-IFN$\gamma$ or anti-TNF$\alpha$ mAb ameliorates disease, and treatment with anti-IL-2 mAb converts acute GVHD to autoimmune GVHD (Via and Finkelman, 1993, *hit Immunol* **5**: 565-572).

Clinical trials of native and recombinant IL-2 in treating HIV-infected patients have been in progress since 1983 (Volberding et al, 1987, *AIDS Res Hum Retroviruses*, **3**: 115-124). Here, the relationship comes from the fact that development of AIDS has been reported to be associated with a shift in the pattern of lymphokines produced (Clerici and Shearer, 1994, *Immunol Today* **15:** 575-581). Over time, in an infected individual progressing towards disease, a decreased expression of Th1 lymphokines such as IL-2 occurs (Maggi et al, 1987, *Eur J Immunol* **17:** 1685-1690, Gruters et al, 1990, *Eur J Immunol* **20**: 1039-1044, Clerici et al, 1993, *J Clin Invest* **91**: 759-765), concomitant with an increased production of Th2 lymphokines such as IL-4 and IL-10 (Clerici et al, 1994, *J Clin Invest* **93**: 768-775, Hoffman et al, 1985, *Virology* **147**: 326-335). T-cells from asymptomatic or long term survivors treated with IL-2 enhanced their anti-HIV activity whereas exposure to IL-4 or IL-10 reduced their ability to suppress HIV replication and to produce IL-2 (Barker et al, 1995, *Proc Soc Nat Acad Sci USA* **92**: 11135-11139).

These current immunomodulatory therapeutics (mAbs and recombinant cytokines) are, however, not without

limitations. For example with chronic monoclonal antibody treatment, the host animal develops antibodies against the monoclonal antibodies thereby limiting their usefulness. 'Humanized' monoclonal antibodies have been developed which apparently reduces the risk of an induced immune response to these mAbs. However, these are still under development, and in addition these new mAbs remain large proteins and therefore may have difficulty reaching there target sites. Cytokine-based therapeutics also have limitations. For example, IL-12 treatment of autoimmune GVHD leads to the development of acute GVHD in mice.

Ribavirin® (1-b-D-ribofuranosyl-1,2,4-triazole-3-carboxamide) is a synthetic nucleoside capable of inhibiting RNA and DNA virus replication (Huffman et al, 1973, *Antimicrob. Agents Chemother* **3**: 235, Sidwell et al, 1972, *Science* **177**: 705). We have confirmed the observations of others who suggested that Ribavirin®, in addition to its antiviral activity, has an effect on certain immune responses (reviewed Jolley and Suchil, 1984, Clinical Applications of Ribavirin®: p93-96). We have also confirmed observations of others that Ribavirin® affects the proliferation of mitogen- and antigen-activated T and B lymphocytes, (Tam et al, 1995 (data not shown), Peavy et al, 1980, *Infection and Immunity* **29**: 583-589) and then when combined with cyclosporin, Ribavirin® showed efficacy in long term allograft survival , Jolley et al (1988, *Transplantation Proc* **20**: 703-706).

In addition, we have significantly advanced the prior research by demonstrating that Ribavirin® modulates the cytokine pattern of an immune response at least in part by promoting a Th1 response and suppressing a Th2 response. In hindsight, this discovery is not inconsistent with prior research. First, Ribavirin® is known to inhibit both functional humoral immune responses, (Peavy et al, 1981, *J Immunol* **126**: 861-864, Powers et al, 1982, *Antimicrob Agents Chemother* **22**: 108-114) and IgE-mediated modulation of mast cell secretion (Marquardt et al, 1987, *J Pharmacol Exp Therapeutics* **240**: 145-149, (both Th2 lymphokine-mediated events). Second, Ribavirin® antagonizes the antiviral effect of azidothymidine (AZT) in peripheral blood lymphocytes from HIV patients (Vogt et al, 1987, *Science* **235**: 1376-1379). This finding is significant because AZT decreases IL-2 receptor (IL-2R) but not IL-2 expression (Viora and Camponeschi, 1995, *Cell Immunol* **163**: 289-295). It is therefore possible that Ribavirin® antagonizes AZT by modulating IL-2 expression and elevating depressed levels of IL-2R. Third, Ribavirin® treatment of an immuno-compromised patient for chronic GVHD (a Th2-mediated disorder) led to a dramatic resolution of the disease, an outcome which did not occur with conventional immunosuppressive therapies such as cyclosporin and glucocorticoids (Cassano, 1991, *Bone Marrow Transplantation* **7**: 247-248). Finally, Ribavirin® treatment (one year) of patients for hepatitis C (HCV) revealed fewer lymphocyte aggregates and far less liver damage than placebo controls (Dusheiko et al, 1994, *Hepatology* **20**: 206A). This observation may reflect the fact that although, the predominant immune response to hepatitis C is mediated by Th1 lymphokines, T cells of the Th0/Th2 phenotype can be infected by HCV (Zignego et al, 1994, unpublished data) and this infection may drive further antibody-mediated destruction of hepatocytes.

## BRIEF DESCRIPTION OF THE DRAWINGS

TABLE 1 represents the resting and PMA/ionomycin-activated levels, at 48 and 72h, of the lymphokines, IL-2, IL-4, TNF$\alpha$ and IFN$\gamma$ (pg/ml) measured in extracellular supernatants and the cell surface expression of IL-2 (IL-2R) and IL-4 (IL-4R) receptors (mean channel fluorescence intensity) from human T cells.

FIGURE 1 is a graphical representation of the effect of Ribavirin® and interferon alpha on the extracellular expression of IL-2, IL-4, TNF$\alpha$ and IFN$\gamma$ in PMA/ionomycin-activated T lymphocytes. Results are expressed as percentage of the increased lymphokine expression following PMA/ionomycin treatment alone.

FIGURE 2 is a graphical representation of the effect of 2, 10 or 50 mM Ribavirin® in the presence of 2000 U/ml interferon alpha (left panels) and the effect of 500, 1000 or 2000 U/ml interferon alpha (right panels) in the presence of 10 mM Ribavirin® on the extracellular expression of IL-2 (A and C) and IL-4 (B and D) in PMA/ionomycin-activated T lymphocytes.

FIGURE 3 is a graphical representation of the effect of Ribavirin® and interferon alpha on IL-2, IL-4 and IFN$\gamma$ mRNA expression in PMA/ionomycin-activated T lymphocytes.

FIGURE 4 is a graphical representation of the effect of Ribavirin® and interferon alpha on the cell surface expression of IL-2 and IL-4 receptors in PMA/ionomycin-activated T lymphocytes. Results arc expressed as percentage of the increased lymphokine receptor expression following PMA/ionomycin treatment alone.

FIGURE 5 is a graphical representation of the expression of intracellular IL-2 expression in resting (A and E) or activated $CD4^+$ (top panel) or $CD8^+$ (bottom panel) T cells treated with PMA/ionomycin alone (B and F) or in the presence of 10 mM Ribavirin® (C and G) or 5000 U/ml interferon alpha (D and H). Data from one experiment is shown and represented as the percentage of cells showing double positive staining for IL-2 and CD4 or CD8.

FIGURE 6 is a graphical representation of contemplated Ribavirin® analogs.

FIGURE 7 is a collection of graphs showing the results of various concentrations of Ribavirin® analogs on IL-2, TNF-$\alpha$, IFN-$\gamma$, IL-4 and IL-5.

SUMMARY OF THE INVENTION

In accordance with one aspect of the present invention, the nucleoside, Ribavirin®®, is administered to a patient in a dosage range which is effective to modulate lymphokine expression in activated T cells. In particular, Ribavirin®® is used to suppress Th2-mediated T cell responses and promote Th1-mediated T cell response.

Thus, instead of administering Ribavirin®® in its well-recognized role as an antiviral agent, Ribavirin® is herein used in the treatment of imbalances in lymphokine expression. Such imbalances may be found to be concomitants of allergic atopic disorders such as allergic asthma and atopic dermatitis, helminth infection and leishmaniasis, and various primary and secondary immunodeficiencies, which may or may not also be associated with viral infection.

In accordance with other aspects of the present invention, one or more Ribavirin® analog is administered to a patient in a dosage range which is effective to modulate lymphokine expression in activated T cells. The Ribavirin® analog(s) may be used to suppress or enhance Th1 or Th2-mediated T cell responses.

DETAILED DESCRIPTION OF SPECIFIC EMBODIMENTS

In a preferred embodiment, Ribavirin® is administered orally to a human patient in a dosage which achieves a blood serum level averaging 0.25 - 12.5 mg/ml, and most

DETAILED DESCRIPTION OF SPECIFIC EMBODIMENTS

In a preferred embodiment, ribavirin is administered orally to a human patient in a dosage which achieves a blood serum level averaging 0.25 - 12.5 µg/ml, and most preferably, approximately 2.5 µg/ml. In a typical individuals, this optimimum serum level, works out to be approximately 4.5 mg/kg/day of body weight which can be administered in doses from 200 - 1200 mg. Preferably the dosages are divided into a number of smaller doses which are then administered throughout the day.

Since ribavirin has been on the market for several years, many dosage forms and routes of administration are known, and all appropriate dosage forms and routes of administration may be utilized. For example, in addition to oral administration, ribavirin may given intravenously, intramuscularly, intraperitoneally, topically, and the like, all of which are known. Pharmaceutical formulations comprising ribavirin may also comprise one or more pharmaceutically acceptable carrier, which may include excipients such as stabilizers (to promote long term storage), emulsifiers, binding agents, thickening agents, salts, preservatives, solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the ribavirin, its use in the therapeutic compositions and preparations is contemplated. Supplementary active ingredients can also be incorporated into the compositions and preparations.

In addition to the therapeutic uses of the ribavirin contemplated herein, ribavirin may also be used as a laboratory tool for the study of absorption, distribution, cellular uptake, and efficacy.

EXAMPLES

**Cell Lines And T Cell Purification**

Peripheral blood mononuclear cells (PBMCs) were isolated from the buffy coat following Ficoll-Hypaque density gradient centrifugation of 60 ml blood from healthy donors. T-cells were then purified from the PBMCs using Lymphokwik lymphocyte isolation reagent specific for T-cells (LK-25T, One Lambda, Canoga Park CA). An average yield of 40 - 60 x $10^6$ T-cells were then incubated overnight at 37 °C in 20 - 30 ml RPMI-AP5 (RPMI-1640 medium

EXAMPLES

**Cell Lines And T Cell Purification**

Peripheral blood mononuclear cells (PBMCs) were isolated from the buffy coat following Ficoll-Hypaque density gradient centrifugation of 60 ml blood from healthy donors. T-cells were then purified from the PBMCs using Lymphokwik lymphocytc isolation reagent specific for T-cells (LK-25T; One Lambda, Canoga Park CA). An average yield of 40 - 60 x $10^6$ T-cells were then incubated overnight at 37 °C in 20 - 30 ml RPMI-AP5 (RPMI-1640 medium (ICN, Costa Mesa, CA) containing 20 mM HEPES buffer, pH 7.4, 5 % autologous plasma, 1 % L-glutamine, 1 % penicillin/streptomycin and 0.05 % 2-mercaptoethanol) to remove any contaminating adherent cells. In all experiments, T-cells were washed with RPMI-AP5 and then plated on 96-well microtitre plates at a cell concentration of 1 x $10^6$ cells/ml.

**T-Cell Activation And Ribavirin® Treatment**

T-cells were activated by the addition of 500 ng ionomycin and 10 ng phorbol 12-myristate 13-acetate (PMA) (Calbiochem, La Jolla, CA) and incubated for 48 - 72h at 37 °C. PMA/ionomycin-activated T-cells were treated with 0.5 - 50 mM Ribavirin® or with 250 - 10000 U/ml of a control antiviral, interferon-alpha (Accurate, Westbury, NY) immediately following activation and re-treated 24 h later. T-cells from each plate were used for immunofluorescence analysis and the supernatants used for extracellular cytokine measurements. Following activation, 900 ml cell supernatant from each microplate was transferred to another microplate for analysis of cell-derived cytokine production. The cells are then used in immunofluorescence analyses for intracellular cytokine levels and cytokine receptor expression.

**Extracellular Cytokine Analyses**

Cell-derived human cytokine concentrations were determined in cell supernatants from each microplate. Activation-induced changes in interleukin-2 (IL-2) levels were determined using a commercially available ELISA kit (R & D systems Quantikine kit, Minneapolis, MN) or by bioassay using the IL-2-dependent cell line, CTLL-2 (ATCC, Rockville, MD). Activation -induced changes in interleukin-4 (IL-4), tumor necrosis factor (TNFα) interleukin-8 (IL-8) (R & D systems (Quantikine kit, Minneapolis, MN) and interferon-gamma (IFNγ) (Endogen (Cambridge, MA) levels were determined using ELISA kits. All ELISA results were expressed as pg/ml and the CTLL-2 bioassay as counts per minute representing the IL-2-dependent cellular incorporation of $^3$H-thymidine (ICN, Costa Mesa, CA) by CTLL-2 cells.

**Direct Immunofluorescence Studies (Cytokine Receptors)**

For direct staining with fluorescence-conjugated antibodies to cell surface antigens, the cells were washed twice with isotonic saline solution, pH 7.4 (Becton Dickinson, Mansfield, MA) and re-suspended in 50 ml isotonic saline solution and split into two samples. One sample aliquot was co-stained with either PE-anti CD25/FITC-anti CD4 or PE-rat anti mouse IgG + anti-CDw124/FITC-anti CD4 mAb and non-specific fluorescence was assessed by staining the second aliquot with PE/FITC-labeled isotype-matched control monoclonal antibody. All fluorescence-labeled monoclonal antibodies were obtained from Becton Dickinson (San Jose, CA) except for anti-CDw124 which was obtained from Pharmingen, San Diego, CA. Incubations were performed at 4 °C in the dark for 45 min using saturating mAb concentrations. Unincorporated label was removed by washing in PBS prior to the analysis with a FACScan flow cytometer (Becton Dickinson).

Antigen density was indirectly determined in gated live $CD4^+$T cells and expressed as the mean channel of fluorescence (MCF). Surface expression of specific antigen (CDw124, CD25) was represented as the mean channel shift (MCS) obtained by subtracting the MCF of FITC- or PE-labeled isotype-matched (IgG1) control mAb-stained cells from the MCF of FITC- or PE-labeled antigen-specific mAb stained cells. Alternatively, surface expression of the $CD4^+$-subset of cells stained with CD28 mAb was determined by subtracting the MCF of $CD28^+$ $CD4^+$ from the MCF of CD28-CD4- cells.

The viability of control untreated and Ribavirin® and interferon a-treated cells were determined in each batch of all oligonucleotides in multiple donors by staining with the vital dye, propidium iodide (5 mg/ml final concentration). The percentage of live cells which excluded propidium iodide was determined by flow cytometry and was > 90 % (range 90 - 99 %) following treatment with all concentrations used.

**Immunofluorescence Analyses of Intracellular Cytokine Expression**

For analyses of the intracellular expression of IL-2 in $CD4^+$ and $CD8^+$ T cell subsets, T cells were first treated for the last 4 h of 48- 72h activation with 10 mg Brefeldin A (Gibco BRL, Gaithersburg, MD) to minimize secretion of newly synthesized IL-2 into the extracellular milieu. Following activation, 900 ml cell supernatant from each microplate was transferred to another microplate for analysis of cell-derived cytokine production. Prior to direct staining (30 min, 4 C , in the dark) with FITC-conjugated antibodies to the cell surface antigens, CD4 and CD8, the cells were washed twice with isotonic saline solution, pH 7.4 and resuspended in 100 - 150 ml Staining Buffer (phosphate buffered saline, pH 7.4 containing 1% Fetal Calf Serum (FCS) (Hyclone, Logan, UT) and 0.1% sodium azide), and split into two samples. Stained cells were washed in 1 ml Staining Buffer and cell pellet resuspended in 100 ml Fixation Buffer (4% paraformaldehyde in PBS) following aspiration of the supernatant. Fixed cells were kept at 4 C for 20 mins, then washed in 1 ml Staining Buffer and cell pellet resuspended with mixing in 50 ml Permeabilization Buffer (0.1% saponin (ICN, Costa Mesa, CA) in PBS). Permeabilized cells were stained with PE-labeled IL-2 antibody for 30 min at 4 C in the dark and then washed in 1 ml Permeabilization Buffer, re-suspended in 250 ml Staining Buffer prior to FACS analysis.

**Analysis of Cytokine mRNA**

Total RNA was extracted from resting T cells and from Ribavirin® and interferon a-treated and untreated activated T cells using a commercial variation of the guanidium thiocyanate/ phenol extraction technique (Trizol reagent (GIBCO/BRL). RNA was washed with 70% ethanol and finally resuspended in 10 μl DEPC-treated water.

cDNA synthesis reaction was performed as per manufacturers instructions (Promega, Madion, WI). Briefly, 1 μg of total RNA was heated at 65° C for 10 min and cooled on ice before combining with 2 μl 10X reverse transcription buffer (100 mM Tris HCl (pH 8.8), 500 mM KCl, 1% Triton X-100), 5 mM MgCl, 2 μl 10 mM dNTPs (1 mM each dNTP), 0.5 μl RNase inhibitor, 1 μl oligo $(dT)_{15}$ primer (0.5 μg/μg RNA) and 0.65 μl AMV reverse transcriptase (H. C.). The reaction was incubated at 42°C for 1 h followed by at 95°C for 10 min and 5 min on ice.

The PCR reaction was performed using GeneAmp PCR kit (Perkin-Elmer Cetus, Foster City, CA). In a fresh tube, RT reaction mixture (3 μl) was combined with 5 μl 10 X PCR buffer ( 500 mM KCl, 100 mM Tris-HCl, pH 8.3, 15 mM $MgCl_2$ and 0.01% (w/v) gelatin), 1 μl 10 mM dNTPs and 1 U of Taq DNA polymerase. The primers used were as follows: interleukin-2, interleukin-4, interferon-g (human) primers (Stratagene, La Jolla, CA) and pHE7 ribosomal gene. Amplification conditions were 45 sec at 94°C, 1 min at 57°C and 2 min at 72°C for 35 cycles, followed by 8 min at 72°C. PCR products were analyzed on 2% agarose gel containing ethidium bromide. Following electrophoresis, PCR products were transferred to Hybond N+ membrane (Amersham, Arlington Heights, IL) in 20 X SSC overnight and immobilized using 0.4 M NaOH. Blots were hybridized with $^{32}$P-γATP labeled oligonucleotide probes in Rapid - hyb buffer (Amersham) for 1 h at 42° C. Each cytokine primer mix was used as a radiolabeled probe (as per instructions). Equivalent loading was assessed following hybridization with a probe generated from pHE7 sense primer. Washed blots were then analyzed using PhosphorImager.

**Effect of Ribavirin® on Extracellular Cytokinc Levels in Activated T Cells**

PMA/ionomycin treatment (48 - 72h) of human T-cells substantially increased the levels of all the cytokines analyzed i.e. IL-2, IL-4, TNFα, IFNγ (TABLE 2). The first number in each cell depicts the arithmetic mean, and the numbers in parenthesis depicts the relevant ranges. N = 4. In a representative experiment shown in Figure 1, addition of Ribavirin®, in the dose range 0.5 - 50 mM, augmented activated levels of the Th1 cytokines, IL-2 and TNFα maximally at 5 mM (30%) and 20 mM (36%) respectively. In contrast, interferon-a, inhibited IL-2 and TNFα expression in a dose-depcndent manner (range 250 - 10000 U/ml, maximal inhibition 33 and 38% respectively), when compared to levels in untreated activated T cells. In addition, Ribavirin® mediated a concomitant decrease in activated levels of the Th2 cytokine, IL-4 (peak inhibition of 74% at 2 mM) whereas interferon-a maximally increased extracellular IL-4 by 26% (10000 U/ml). Using combinations of Ribavirin® and interferon alpha, Figure 2 shows that a constant 2000 U/ml of interferon alpha suppressed the Ribavirin® dose-dependent augmentation of activated IL-2 levels (A) and reversed the inhibition of activated IL-4 levels (C). Similarly, a constant 10 mM of Ribavirin® reversed the interferon alpha-mediated dose-dependent inhibition of activated IL-2 levels (B) and suppressed the augmentation of activated IL-4 levels (D).

**Effect of Ribavirin® on Cytokine mRNA Levels in Activated T Cells**

These opposing effects of Ribavirin® and interferon-a on activated extracellular cytokine levels were also observed at the level of transcription. Figure 3 shows that PMA/ionomycin treatment of human T-cells substantially augments IL-2, IL-4 and IFNγ mRNA levels. Treatment with Ribavirin® (2, 5 and 10 mM) following T cell activation, elevates IL-2, decreases IL-4 and has no effect on IFNγ mRNA. In contrast, interferon a, at 1000, 2000 and 5000 U/ml decreases IL-2, increases IL-4 and decreases IFNγ mRNA. Therefore the respective dose-dependent effects of Ribavirin® and interferon a on IL-2, TNFα, and IL-4 mRNA expression paralleled the ELISA analyses. These data suggest that Ribavirin® promotes the synthesis of the Th1 cytokines, IL-2 and TNFα and inhibits the expression of the Th2 cytokine, IL-4 in activated human T cells

**Effect of Ribavirin® on IL-2 and IL-4 Receptor Levels in Activated T Cells**

Using FACS analysis, we compared the effects of Ribavirin® and interferon a on expression of IL-2 (CD25) and IL-4 (CDw124) receptor expression in activated T cells. PMA/ionomycin-treatment increases CD25 and CDw124 expression from resting levels of 50.16 ± 0.45 and 62.31 ± 1.46 to activated levels of 162.48 ± 2.89 and 87.53 ± 3.98 respectively (n = 4). In a representative of 3 experiments, Figure 4 show that Ribavirin® (1 - 50 mM) has little effect on activated levels of IL-2 and IL-4 receptor whereas interferon a, in the dose range 250 - 10000 U/ml, decreased IL-2 receptor and increased IL-4 receptor expression in a dose-dependent manner, when compared to receptor levels in control activated T cells. Therefore, these data show that the effect of Ribavirin® on cytokine synthesis acts independently of cytokine receptor expression. In contrast, the effect of interferon a treatment on IL-2 and IL-4 receptor correlates with that observed with its effect on activated IL-2 and IL-4 expression.

**Effect of Ribavirin® on Intracellular IL-2 Levels in CD4 and CD8+ Subsets of Activated T Cells**

We examined whether the effect of Ribavirin® on IL-2 expression was specific to $CD4^+$ or $CD8^+$ T cells. Intracellular IL-2 expression in fixed and Permeabilized activated T cells was determined by two-color flow cytometry using fluorescence-labeled antibodies to CD4 or CD8 and to IL-2. Figure 5 shows that following treatment with Ribavirin® at 10 mM, the percentage of $CD4^+$ T cells expressing IL-2 rose from 82 to 91% and the percentage of $CD8^+$ expressing IL-2 increased from 81 to 91%. In contrast, the percentage of IL-2-expressing $CD4^+$ and $CD8^+$ cells following interferon-a-treatment (5000 U/ml) was 81 and 71% respectively. These data suggest Ribavirin® has an effect on intracellular IL-2 expression which does not discriminate between $CD4^+$ or $CD8^+$ T cell subsets. In contrast, interferon-a-treatment has little effect on $CD4^+$ T cells and even reduces IL-2 expression in the $CD8^+$ T cell subset.

**Ribavirin® Analogs**

There are several classes of Ribavirin® analogs which are contemplated to be clinically effective in modulating Th1 or $Th_2$-mediated T cell responses. This includes molecules falling with the generic formulae of Figure 6, wherein **X** is O, S, $CH_2$, CHOH or N-CO-$R_{11}$; **A, B** and **C** are independently N, P, CH, C-OH, C-$CH_3$, C-alkyl, C-alkenyl, C-$CH_2$, -CN, C-halogen, C-CN, C-$COOCH_3$, C-$NH_2$, C-$SNH_2$, C-$SO_2$-$NH_2$, C-$CONH_2$, C-CS-$NH_2$, C-C(NH)$NH_2$, $CPO_2$-$NH_2$, or C-heterocyclic ring system; **D** is S, Se, Te, PH, NH or $NR_{12}$; $\mathbf{R_1}$ is H, $(CH_2)$p(OH), halogen, CN, $(CH_2)$p$ONH_2$, $(CH_2)$p$NH_2$, $CH_3$, $CH_2$SPH or $(CH_2)$-heterocyclic ring; $\mathbf{R_2}$ is H, OH, $OCH_3$, SH, $SCH_3$, halogen, CN, $NH_2$, $ONH_2$, $NHCH_3$, $(CH_2)$OH, $(CH_2)$p$NH_2$, $CH_3$, or COOMe; $\mathbf{R_3}$ $\mathbf{R_4}$, $\mathbf{R_5}$, $\mathbf{R_6}$, $\mathbf{R_7}$ and $\mathbf{R_8}$ are independently H, OH, $OCH_3$, SH, $SCH_3$, halogen, CN, $NH_2$, $ONH_2$, $NHCH_3$, $(CH_2)$OH, $(CH_2)$p$NH_2$, $CH_3$, COOMe or phenyl; $\mathbf{R_9}$ is H, halogen, $NH_2$, $CH_3$, CONH, $CSNH_2$, COOMe, $SNH_2$, $SO_2NH_2$, $PO_2NH_2$, $(CH_2)$p, $(CH_2)$p-heterocyclic ring system or $(CH_2)$p-glucose; $\mathbf{R_{10}}$ is H, halogen, $NH_2$, $CH_3$, CONH, $CSNH_2$, COOMe, $SNH_2$, $SO_2NH_2$, $PO_2NH_2$, $(CH_2)$p, $(CH_2)$p-heterocyclic ring system, $(CH_2)$p-glucose, O-$CH_3$, O-$CH_2CH_3$ or amino acid; **Y** is O, S, NH•HCl, NOH, $NOCH_3$ or $NOCH_2$PH; $\mathbf{R_{10}}$ **& Y** in combination are a heterocyclic ring systems such as thiazole, imidazole, etc., $\mathbf{R_{11}}$ is $CH_3(CH_2)$p$NH_2$, $(CH_2)$p-heterocycle, $(CH_2)$p-amino acid or $(CH_2)$p-sugar (glucose etc); **p** is an integer between 0 and 8; and the above includes both L and D nucleosides.

Such molecules can be manufactured according to one or more of the following exemplary schemes.

1. Synthesis of 1CN1369 (Pyrazomycin): The synthetic methodology for this compound is described in the following references: (a) J. Farkas, Z. Flegelova and F. Sorm, *Tetrahedron Letts.*, 22, 2279(1972); (b) S. De Bernardo and M. Weigele, J. *Org. Chem.*, 41, 287 (1976); (c) J. G. Buchanan, A. Stobie and R. H. Wightrnan, J. *Chem. Soc. Chem. Commun.*, 916 (1980); N. Karagiri, K. Takashirna, T. Haneda and T. Kato, J. *Chem. Soc. Perkin Trans.!, 553* (1984).
2. Synthesis of 1CN3438 (1-β-D-xylofuranosyl-1,2,4-triazole-3-carboxamide): The synthesis of this compound was accomplished by following the reported procedure described in J. T. Witkowski, M. Fuertes, P. D. Cook and R. K. Robins, J. *Carbohydr. Nucleosides, Nucleotides* 2(1), 1 (1975)).
3. Synthesis of 1CN3844 (1 (5-O-sulfamoyl-β-D-ribofuranosyl)-1,2,4-triazole-3-carboxamide): The compound was prepared by following the procedure disclosed in G. D. Kini, B. M. Henry, R. K. Robins, S. B. Larson, J. J. Marr, R. L. Berens, C. J. Bacchi, H. C. Nathan and 3.S. Keithly, J. *Med.* Chem., 33, 44 (1990).
4. Synthesis of 1CN4625 (1(3-deoxy-β-D-erythropentofuranosyl)-1,2,4-triazole-3-carboxamide): The compound was prepared by utilizing the following synthetic method.

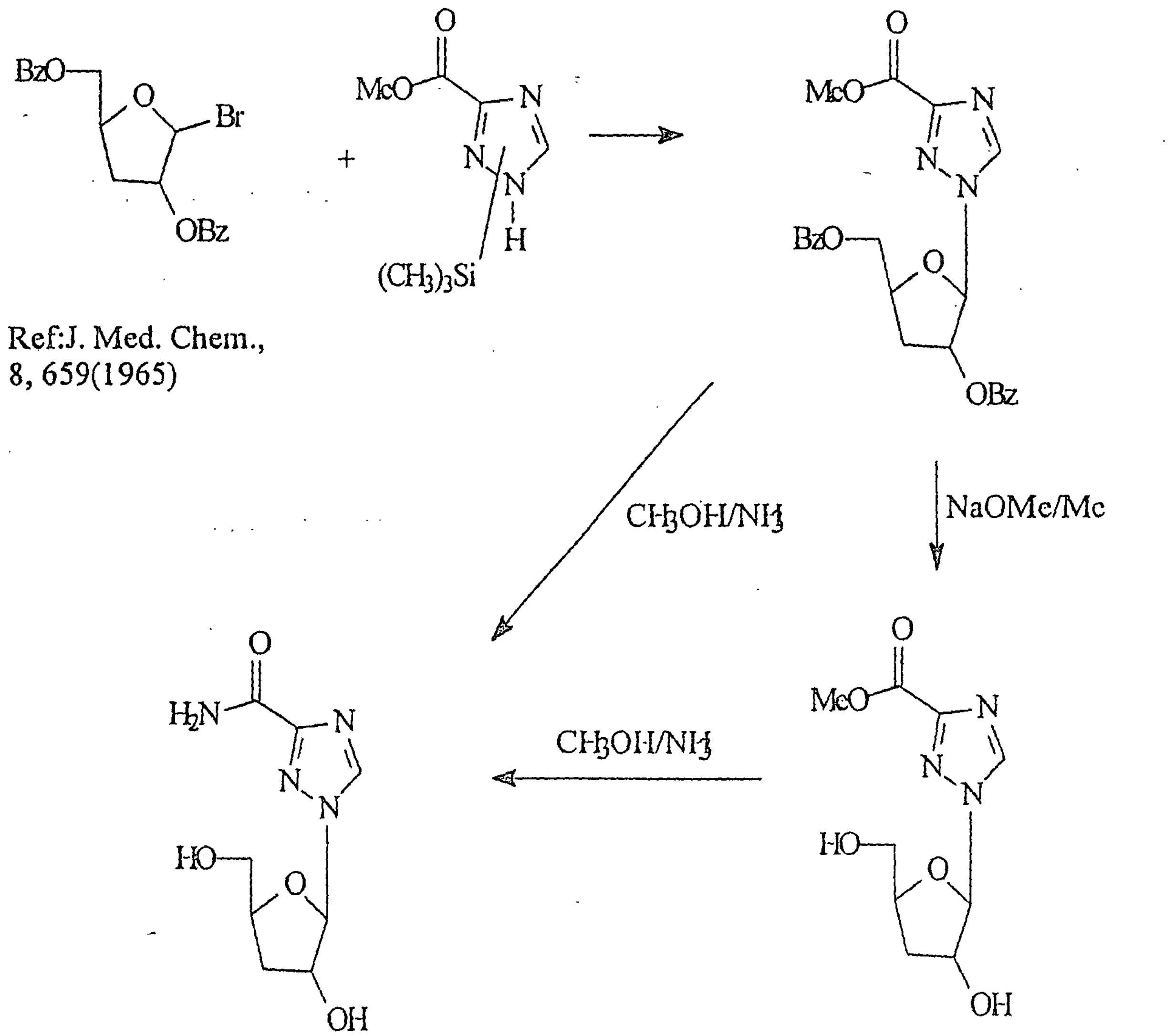


5. Synthesis of ICN5531 (5-amino-1-β-D-ribofuranosylpyrazol-4-carboxamide): The synthetic methodology reported in B. K. Bhattacharya, R. K. Robins and G. R. Revankar, J. *Heterocyclic Chem.*, *21,795* (1990) was followed to prepare the titled compound.

6. Synthesis of 1CN5676 (1-β-D-arabinofuranosyl-1,2,4-triazole-3-carboxamide): The synthesis of this compound was accomplished by following the reported procedure in J. T. Witkowski, M Fuefles, *P.* D. Cook and R. K. Robins, J. *Carbohydr. Nucleosides, Nucleotides*, 2(1), 1 (1975)).

7. Synthesis of ICN5839 (1-β-D-erythrofuranosyl-1,2,4-3-carboxamide): This compound was prepared using the synthetic sequence described below:

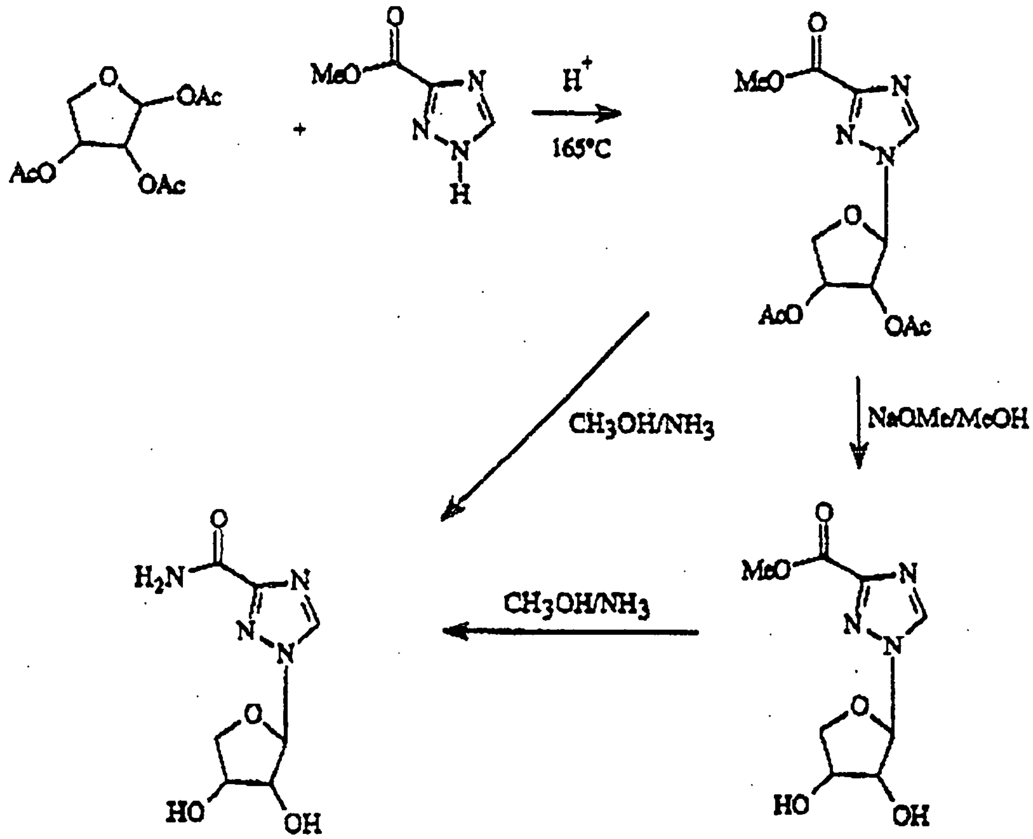


8. Synthesis of ICN6242 (2-bromo-1-β-D-ribofuranosylimidazole-4-carboxamide): 1CN6242 was prepared by following the method shown below.

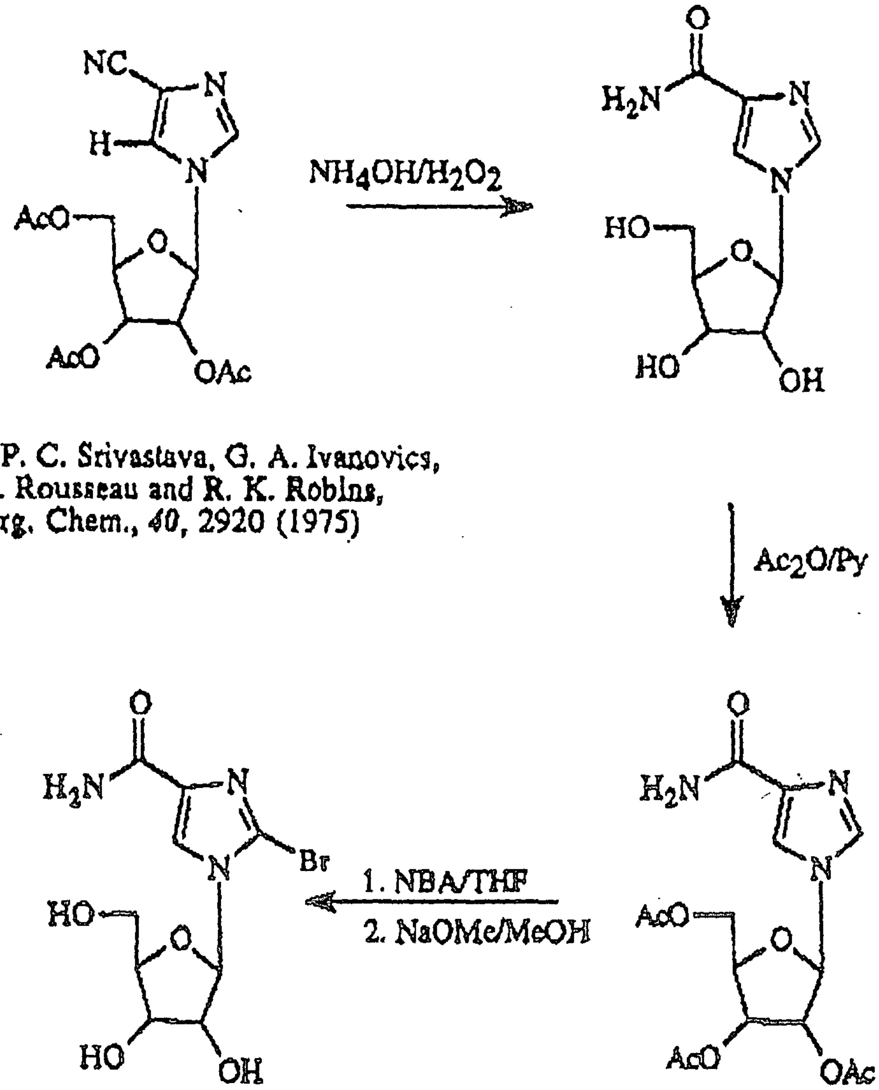


9. Synthesis of ICN11808 (1-β-D-ribofuranosyl-pyrazole-3,4-dicarboxamide): The synthesis of this compound was described in Y. S. Sanghvi, B. K. Bhattacharya, G. D. Kini, S. S. Matsumoto, S. B. Larson, W. J. Jolley, R. K. Robins and G. R. Revankar, J. *Med. Chem.*, 33, 336 (1990) and was prepared accordingly.

10. Synthesis of 1CN 12204 (2-(β-D-ribofuiranosyl)imidazole-5-carboxamide): The synthesis of this compound was achieved by following the reported procedure ii' J. Igoleo, T. H. Dinh, A. Keib and C. Perreur, *Chimie Therapeutique*, 207 (1972).

For the Carbocylic 4'-thiosugar and 4'-azasugar derivatives of the Ribavirin® analogs, the following sugars could be condensed with appropriate het&ocyclic compounds and derivatized as described in the above schemes and references.

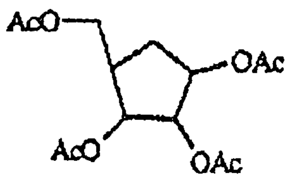

Carbocyclicsugar

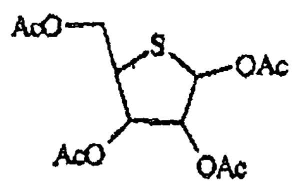

4'-Thiosugar

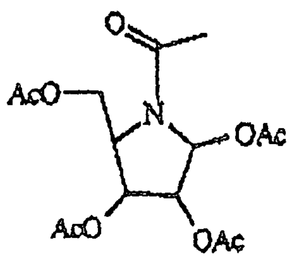

4'-Azasugar

1. The carbocyclic sugars can be prepared by following the literature procedure reported: M. Yoshikawa, Y. Yoshikawa, Y. Inouc, S. Yamaguchi and N. Murakami, Tetrahedron, 50,9961 (1994); L. Agrofoglio, E. Suhas, A. Parese, R. Condom, S. R. Challand, R. A. Earl and R. Guedj, Tetrahedron, 50, 10611 (1994).
2. The 4-azasugar was prepared by the reported literature procedure: E. J. Reist, D. E. Gueffroy and L. Goodman, J. Am. Chem. Soc., 87, 677 (1965).
3. The 4-thio-D-ribofuranose was prepared by following the literature procedure: M. Hobek and R. L. Whistler in "Methods in Carbohydrate Chemistry," Vol 1,292(1962).

Evidence of the effectiveness of the Ribavirin® analogs in modulating TH1 and Th2 cytokines is set forth in Figure 7 and in the table below. The assays reported here comprise a short list of 35 Ribavirin® analogs tested, and all assays were performed three times. In general, it can be seen that the compounds tested fall into three major categories: (1) compounds such as ICN1369 and ICN3844 which suppressed IFNγ, IL-2, IL-4, IL-S and TNFα; (2) compounds such as Ribavirin® which enhanced IL-2 and TNFα and suppressed ILA and IL-5; and (3) compounds such as ICN6242, ICN3839 and ICN3531 which enhanced IL-2 and IFNγ and suppressed IL-4 and IL-5. Such activities listed above would be potentially beneficial in indications in which modulation of cytokine would impact an appropriate immune response.

TABLE 2

| | 1 | 2 | 3 | 4 | 5 | 6 | |
|---|---|---|---|---|---|---|---|
| A | Media | PMA<br>ION | PMA<br>ION | PMA<br>ION<br>10uM 1369 | PMA<br>ION<br>5uM 1369 | PMA<br>ION<br>2uM 1369 | A |
| B | PMA<br>ION<br>10uM 3438 | PMA<br>ION<br>5uM 3438 | PMA<br>ION<br>2uM 3438 | PMA<br>ION<br>10uM 3844 | PMA<br>ION<br>5uM 3844 | PMA<br>ION<br>2uM 3844 | B |
| C | PMA<br>ION | PMA<br>ION | PMA<br>ION | PMA<br>ION | PMA<br>ION | PMA<br>ION | C |

TABLE 2 (continued)

| | 1 | 2 | 3 | 4 | 5 | 6 | |
|---|---|---|---|---|---|---|---|
| | 10uM 4625 | 5uM 4628 | 2uM 4615 | 10uM 8531 | 5uM 5531 | 2uM 5531 | |
| D | PMA<br>ION<br>10uM 5676 | PMA<br>ION<br>5uM 5676 | PMA<br>ION<br>2uM 5676 | PMA<br>ION<br>10uM 5839 | PMA<br>ION<br>5uM 5839 | PMA<br>ION<br>2uM 5839 | D |

**PLATE 2** Cell concentration por well a 2 x $10^6$ x 12 a 24 x $10^6$ in 30ml (Total volume of each well = 2000 ul)

| | 1 | 2 | 3 | 4 | 5 | 6 | |
|---|---|---|---|---|---|---|---|
| A | PMA<br>ION<br>10uM 6242 | PMA<br>ION<br>5uM 6242 | PMA<br>ION<br>2uM 6242 | PMA<br>ION<br>10uM<br>11808 | PMA<br>ION<br>5uM 11808 | PMA<br>ION<br>2uM 11808 | A |
| A | PMA<br>ION<br>10uM<br>12204 | PMA<br>ION<br>5uM<br>12204 | PMA<br>ION<br>2uM 12204 | PMA<br>ION<br>10uM RIB | PMA.<br>ION.<br>5uM RIB | PMA<br>ION<br>2uM RIB | A |

Thus, Ribavirin® and Ribavirin® analogs have been shown to be effective in modifying lymphokine express in activated T-cells. While specific embodiments and applications have been addressed, it would be apparent to those skilled in the art that many more modifications are possible without departing from the inventive concepts herein. The invention, therefore, is not to be restricted except in the spirit of the appended claims.

According to the invention:

- The use of Ribavirin® to modulate lymphokine expression in activated T cells of a human patient, wherein Ribavirin® is preferably administered in a dose effective to raise the level of Th1 and lower the level of Th2.
- The use of Ribavirin® to treat a non-viral disease characterized by a cytokine profile in which Th1 is reduced below normal and Th2 is increase above normal, wherein the disease may comprise an allergy, wherein the disease may comprise an autoimmune disease, wherein the disease may comprise a helminthic disease.
- The use of Ribavirin® to treat the non-viral component of a disease having a viral component and a non-viral component, the non-viral component being characterized by reduced Th1 levels and increased Th2 levels in activated T-lymphocytes, wherein the disease may involve primary immunodeficiency, wherein the disease may involve secondary immunodeficiency, wherein the viral component may involve the human immunodeficiency virus.
- The use of any of the following compounds in modulating Th1 or Th2 expression:

where:

X is O, S, $CH_2$, CHOH or N-CO-$R_{11}$;

**A**, **B** and **C** are independently N, P, CH, C-OH, C-$CH_3$, C-alkyl, C-alkenyl, C-$CH_2$,-CN, C-halogen, C-CN, C-$COOCH_3$, C-$NH_2$, C-$SNH_2$, C-$SO_2$-$NH_2$, C-$CONH_2$, C-CS-$NH_2$, C-C(NH)$NH_2$, $CPO_2$-$NH_2$, or C-heterocyclic ring system;
**D** is S, Se, Te, PH, NH or $NR_{12}$;
$\mathbf{R_1}$ is H, $(CH_2)$p(OH), halogen, CN, $(CH_2)$p$ONH_2$, $(CH_2)$p$NH_2$, $CH_3$, $CH_2SPH$ or $(CH_2)$-heterocyclic ring;
$\mathbf{R_2}$ is H, OH, $OCH_3$, SH, $SCH_3$, halogen, CN, $NH_2$, $ONH_2$, $NHCH_3$, $(CH_2)OH$, $(CH_2)$p$NH_2$, $CH_3$, or COOMc;
$\mathbf{R_3}$ $\mathbf{R_4}$, $\mathbf{R_5}$, $\mathbf{R_6}$, $\mathbf{R_7}$ and $\mathbf{R_8}$ are independently H, OH, $OCH_3$, SH, $SCH_3$, halogen, CN, $NH_2$, $ONH_2$, $NHCH_3$, $(CH_2)OH$, $(CH_2)$p$NH_2$, $CH_3$, COOMe or phenyl;
$\mathbf{R_9}$ is H, halogen, $NH_2$, $CH_3$, CONH, $CSNH_2$, COOMe, $SNH_2$, $SO_2NH_2$, $PO_2NH_2$, $(CH_2)$p, $(CH_2)$p-heterocyclic ring system or $(CH_2)$p-glucose;
$\mathbf{R_{10}}$ is H, halogen, $NH_2$, $CH_3$, CONH, $CSNH_2$, COOMe, $SNH_2$, $SO_2NH_2$, $PO_2NH_2$, $(CH_2)$p, $(CH_2)$p-heterocyclic ring system, $(CH_2)$p-glucose, O-$CH_3$, O-$CH_2CH_3$ or amino acid;
**Y** is O, S, NH•HCl, NOH, $NOCH_3$ or $NOCH_2PH$;

**$R_{10}$** & **Y** in combination are a heterocyclic ring systems such as thiazole, imidazole;
**$R_{11}$** is $CH_3(CH_2)pNH_2$, $(CH_2)p$-heterocycle, $(CH_2)p$-amino acid or $(CH_2)p$-sugar (glucose etc);
**p** is an integer between 0 and 8;

and the above includes both L and D nucleosides.

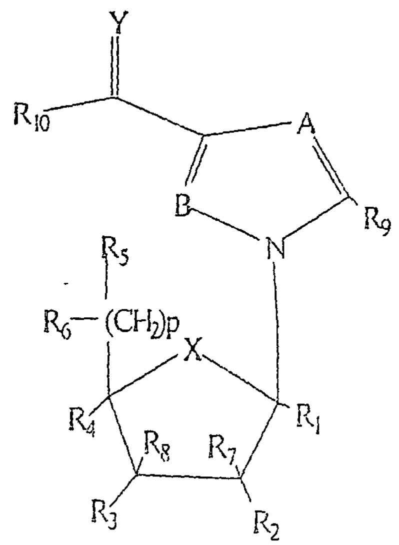

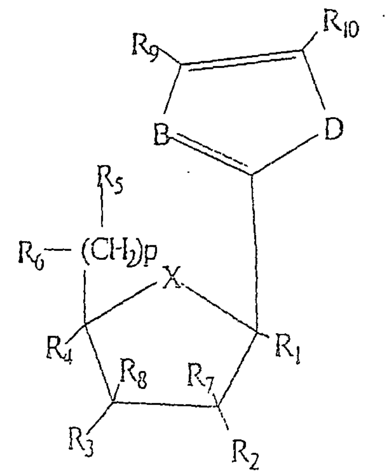

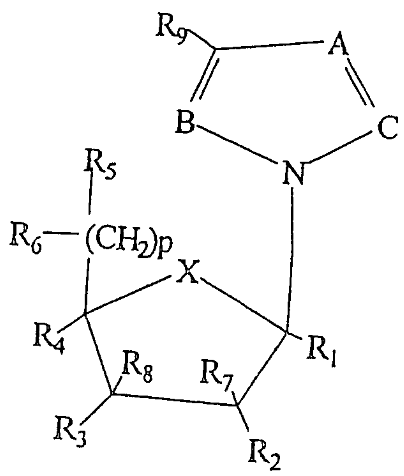

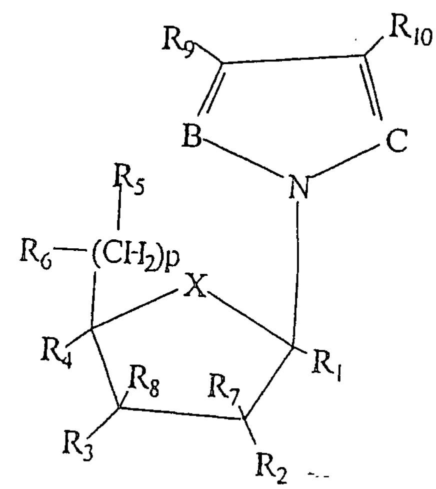

**Claims**

**1.** A method of selling ribavirin comprising:

receiving information that ribavirin may have little or no direct antiviral effect against the HCV virus;
receiving information that ribavirin may be beneficial in treating an HCV infection within a dosage range based on a possible immunomodulatory effect; and
encouraging a physician to administer the ribavirin to treat the HCV infection.

**2.** The method of claim 1 further comprising encouraging the physician to administer the ribavirin in a combination therapy with an interferon.

3. The method of claim 1 wherein it the possible immunomodulatory effect is not clinically proven.

4. The method of claim 1 further comprising encouraging the physician to administer the ribavirin in a combination therapy with an interferon.

5. The method of claim 1 wherein it the possible immunomodulatory effect is not clinically proven.

6. A method of treating a Hepatitis C Virus (HCV) infection in a patient comprising:

receiving information that ribavirin may have little or no direct antiviral effect against the HCV virus;
receiving information that ribavirin may be beneficial in treating the HCV infection within a dosage range based on a possible immunomodulatory effect; and
encouraging a physician to administer the ribavirin to treat the HCV infection.

7. The method of claim 6 further comprising encouraging the physician to administer the ribavirin in a combination therapy with an interferon.

8. The method of claim 6 wherein it the possible immunomodulatory effect is not clinically proven.

9. A method of selling Ribavirin to a physician for administering to a patient in a dosage range in order to induce an increase in Th1 activity in the patient thereby effecting a favorable immunological response.

10. The method of claim 1 further comprising provision of an interferon to be administered in combination with the Ribavirin.

# Table 1

| | 48h Resting | 48h Activated | 72h Resting | 72h Activated |
|---|---|---|---|---|
| IL-2 | 6.7 (5 - 9.3) | 1652 (848 - 2148) | 5.8 (5 - 9.3) | 1462 (918 - 1866) |
| IL-4 | 7.5 (7.1 - 8.2) | 209 (81 - 363) | 8.5 (7.2 - 9.2) | 131 (121 - 148) |
| TNFα | 11.5 (5 - 18) | 1573 (1474 - 1672) | 8.3 (5 - 12) | 1894 (1240 - 2548) |
| IFNγ | 8.9 (8.3 - 9.5) | 1285 (807 - 1765) | 9.3 (9.1 - 9.4) | 2229 (1230 - 3228) |
| IL-2R | 50.1 (40.6 - 59.7) | 163 (160.9 - 166.5) | 60.1 (49.8 - 70.4) | 163 (160.1 - 165.5) |
| IL-4R | | | 52.3 (42.6 - 59.2) | 77.7 (73.5 - 82.1) |

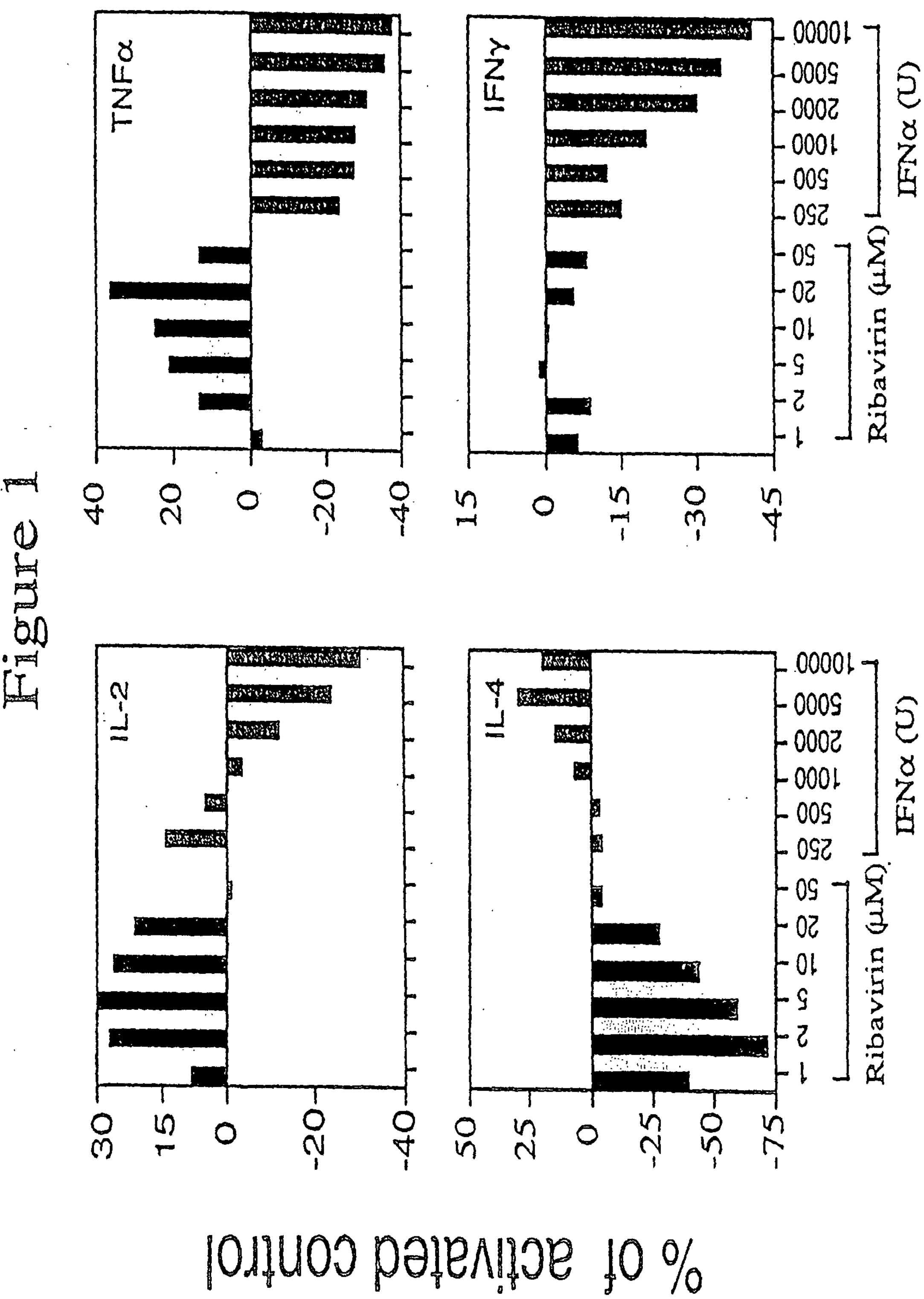
TNFα
IFNγ
IL-2
IL-4
% of activated control
Ribavirin (μM)
IFNα (U)
1
2
5
10
20
50
250
500
1000
2000
5000
10000
40
20
0
-20
-40
15
-15
-30
-45
30
50
25
-25
-50
-75

Figure 1

Figure 2

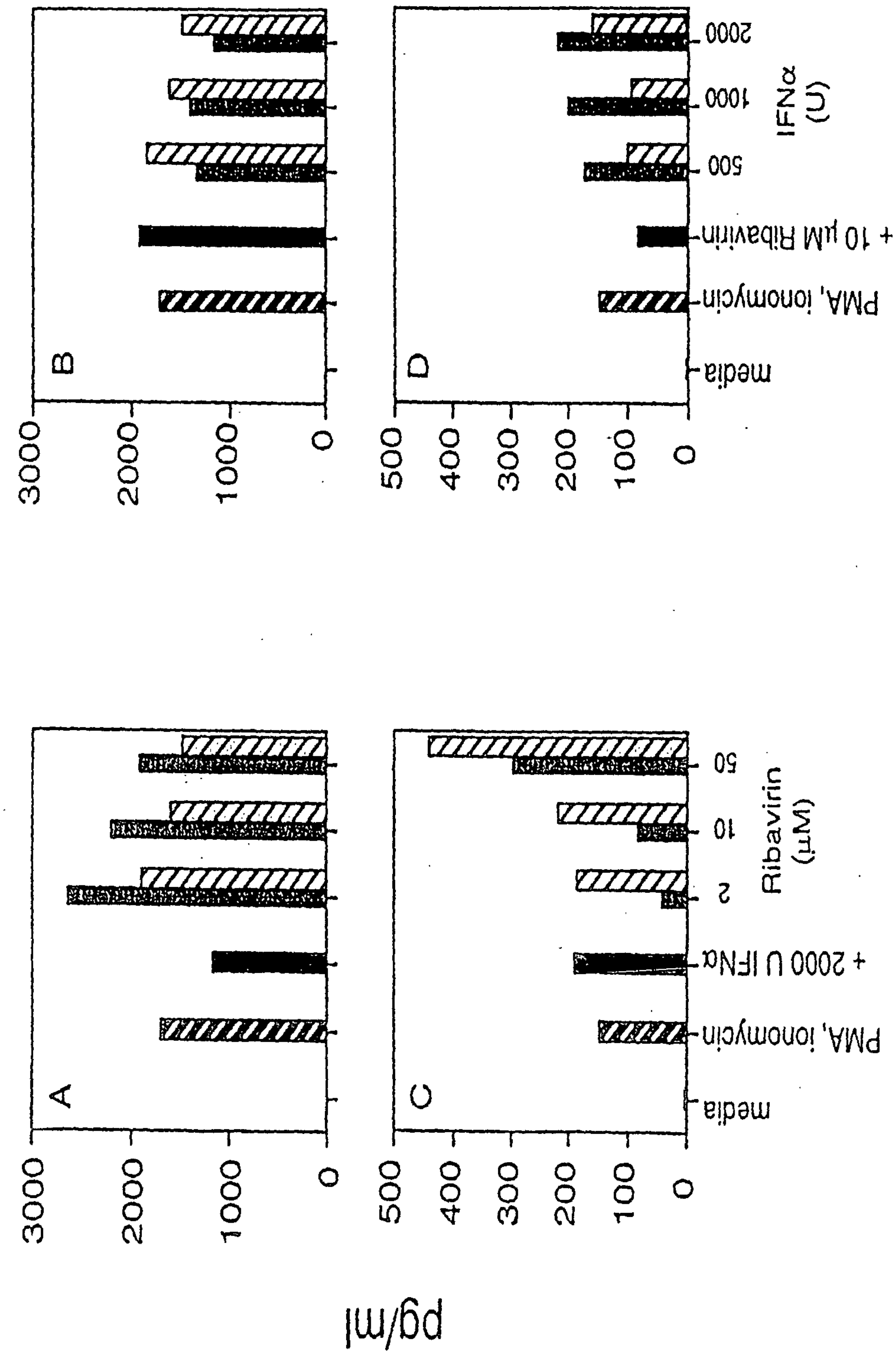
A
B
C
D
pg/ml
3000
2000
1000
0
500
400
300
200
100
0
media
PMA, ionomycin
+ 2000 U IFNα
2
10
50
Ribavirin
(μM)
+ 10 μM Ribavirin
500
1000
2000
IFNα
(U)

Figure 3

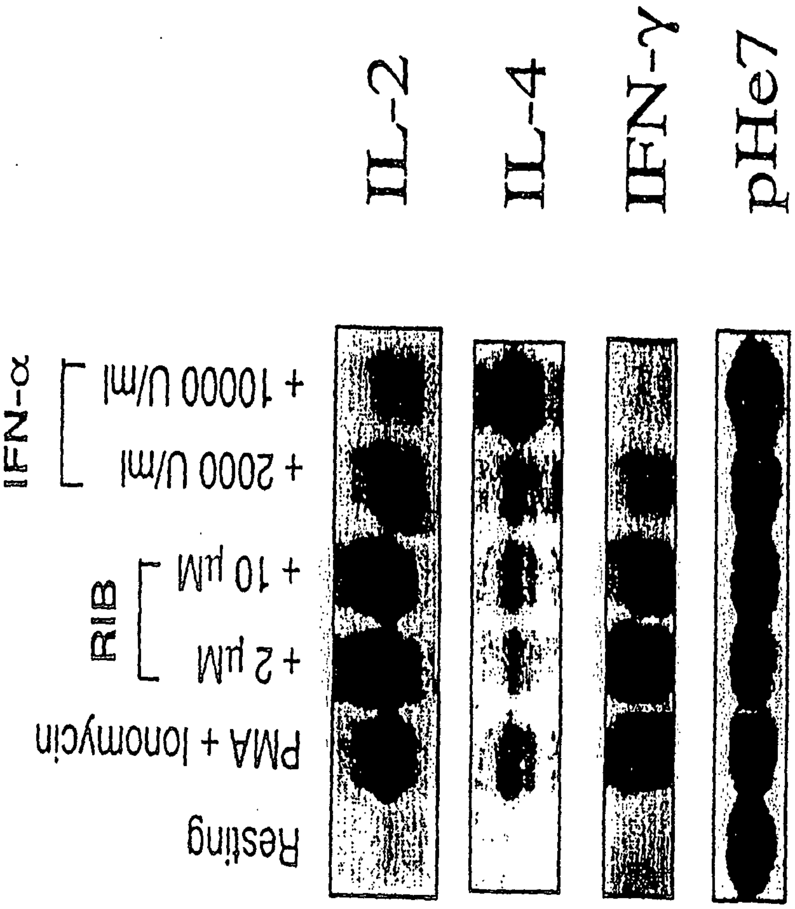
Resting
PMA + Ionomycin
RIB
+ 2 µM
+ 10 µM
IFN-α
+ 2000 U/ml
+ 10000 U/ml
IL-2
IL-4
IFN-γ
pHe7

Figure 4

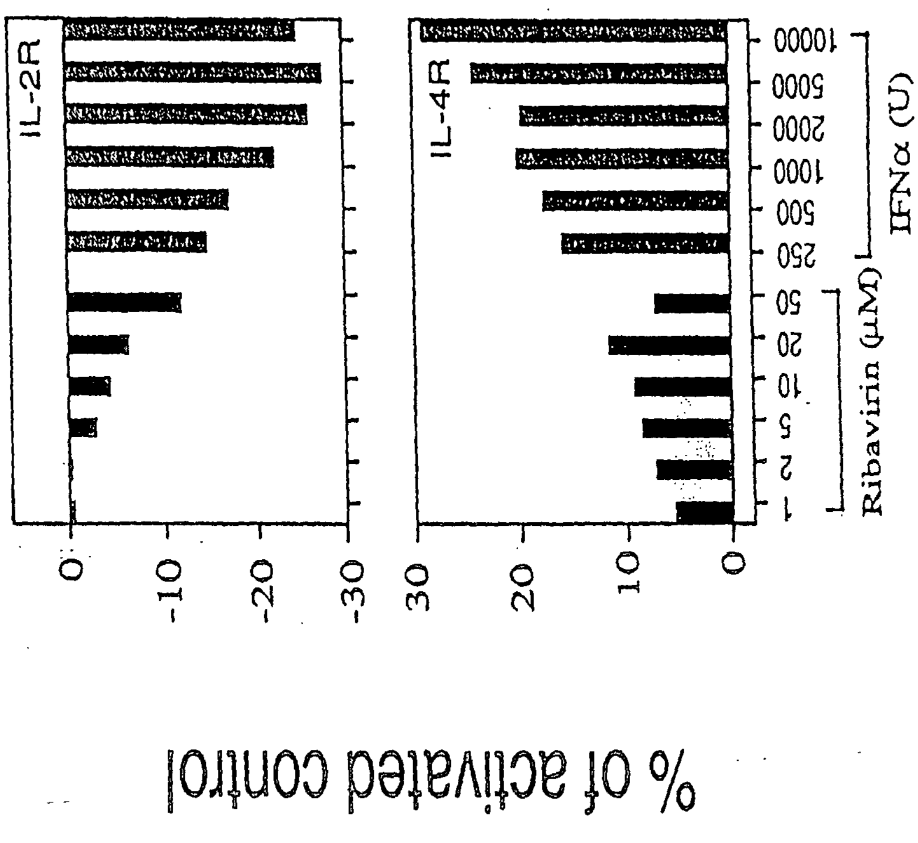
IL-2R
IL-4R
0
-10
-20
-30
30
20
10
0
1
2
5
10
20
50
250
500
1000
2000
5000
10000
Ribavirin (μM)
IFNα (U)
% of activated control

Figure 5

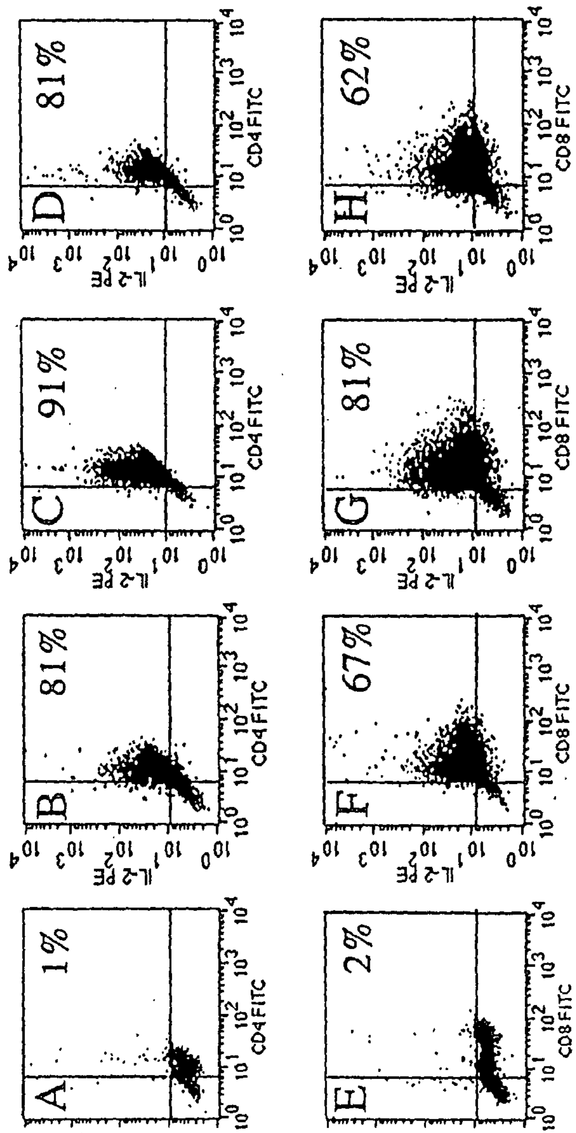

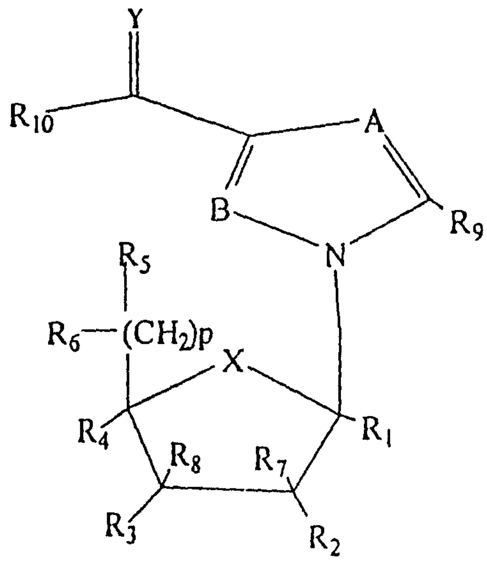

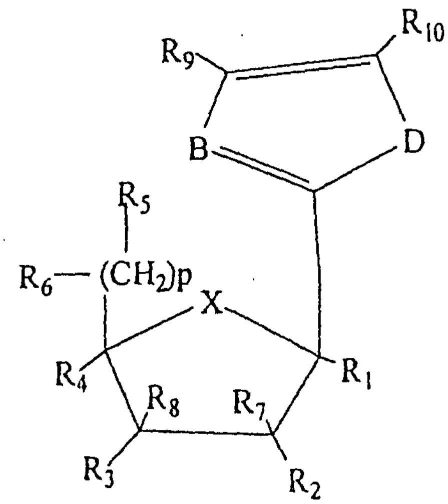

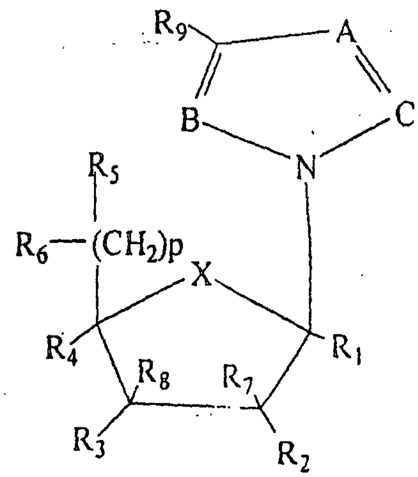

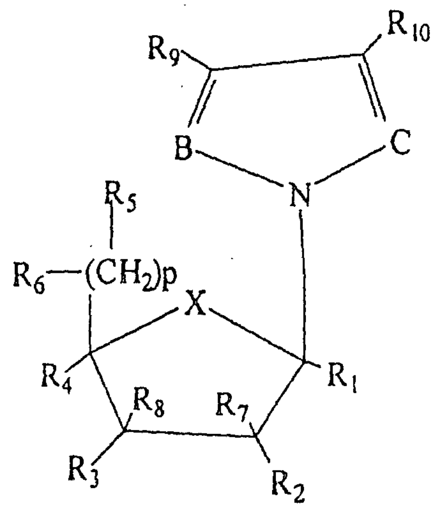

FIGURE 6

ELISA assays from donor TB111 T cell sups from plates setup 11/18/96

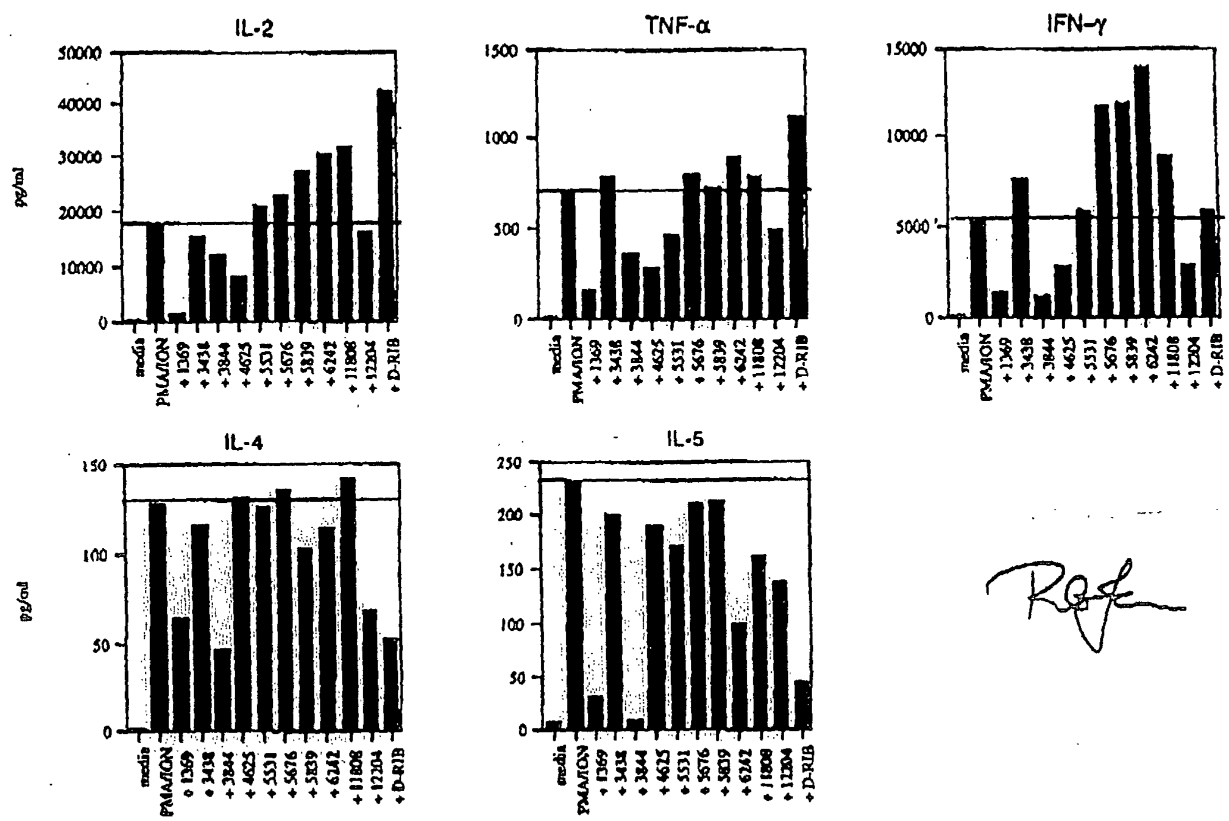


Figure 7